Europäisches Patentamt

European Patent Office

Office européen des brevets

(19)

(11) Veröffentlichungsnummer: **0 004 650**
**B1**

# (12) EUROPÄISCHE PATENTSCHRIFT

(45) Veröffentlichungstag der Patentschrift:
**29.01.86**

(21) Anmeldenummer: **79100968.1**

(22) Anmeldetag: **30.03.79**

(51) Int. Cl.⁴: **A 61 K 31/445**

(54) **Verwendung von 1,4-Dihydro-2,6-dimethyl-4-(3-nitrophenyl)-pyridin-3-beta-methoxy-ethylester-5-isopropylester zur Herstellung von cerebralwirksamen Arzneimitteln.**

(30) Priorität: **11.04.78 DE 2815578**

(43) Veröffentlichungstag der Anmeldung:
**17.10.79 Patentblatt 79/21**

(45) Bekanntmachung des Hinweises auf die Patenterteilung:
**29.01.86 Patentblatt 86/5**

(84) Benannte Vertragsstaaten:
**BE CH DE FR GB IT LU NL SE**

(56) Entgegenhaltungen:
**DE - A - 2 302 866**
**DE - A - 2 407 115**
**GB - A - 1 358 951**

(73) Patentinhaber: **BAYER AG, Konzernverwaltung RP**
**Patentabteilung, D-5090 Leverkusen 1 Bayerwerk (DE)**

(72) Erfinder: **Meyer, Horst, Dr., Theodor-Heuß-Strasse 110,**
**D-5600 Wuppertal 1 (DE)**
Erfinder: **Bossert, Friedrich, Dr., Claudiusweg 7,**
**D-5600 Wuppertal 1 (DE)**
Erfinder: **Kazda, Stanislav, Dr., Pahlkestrasse 55N,**
**D-5600 Wuppertal 1 (DE)**
Erfinder: **Hoffmeister, Friedrich, Prof.Dr.,**
**Katernbergerstrasse 262, D-5600 Wuppertal 1 (DE)**
Erfinder: **Vater, Wulf, Dr., Menchendahlerstrasse 23,**
**D-5090 Leverkusen 3 (DE)**

## Beschreibung

Die Erfindung betrifft die Verwendung von 1,4-Dihydro-2,6-dimethyl-4-(3-nitrophenyl)-pyridin-3-β-methoxyethylester-5-isopropylester zur Herstellung von cerebralwirksamen Arzneimitteln.

Es ist bereits bekannt, dass 1,4-Dihydropyridinderivate als Coronarmittel und Blutdruckmittel verwendet werden können (vgl. DT-OS 2 117 571). Die obengenannte Verbindung ist bereits in der Britischen Patentschrift 1 358 951 des Anmelders als coronarwirksame Verbindung beschrieben. Weiterhin ist bekannt geworden, dass bestimmte basische 1,4-Dihydropyridinester eine cerebrale Wirkung besitzen (vgl. DT-OS 2 302 855 und DT-OS 2 407 115).

Es wurde nun gefunden, dass die Verbindung 1,4-Dihydro-2,6-dimethyl-4-(3-nitrophenyl)-pyridin-3-β-methoxyethylester-5-isopropylester eine sehr vorteilhafte Wirkung auf cerebrale Durchblutungsstörungen besitzt.

Überraschenderweise zeigt die erfindungsgemäss zu verwendende Verbindung in sehr niederen Dosen eine cerebral-spezifische Wirkung, die ihre Verwendung bei der Behandlung cerebraler Insuffizienzen, insbesondere cerebraler Durchblutungsstörungen verschiedener Provenienz ermöglicht. Sowohl in der Wirkungsstärke als auch in der Wirkungsart und Spezifität ist die Verbindung den bekannten cerebralwirksamen Stoffen überlegen.

Sie eignet sich insbesondere für die Behandlung von altersbedingten und sklerotisch beding-ten cerebralen Gefässerkrankungen, sowie cere-bralen Hypoxidosen, posttraumatischen Hirnschä-digungen, allgemeinen Gefäss- und stoffwechsel-bedingten cerebralen Leistungsschwächen, Gleichgewichtsstörungen und anderen vestibulä-ren Erkrankungen und gefässbedingten Sehstö-rungen.

In der Reihe der aus dem Stand der Technik bekannten Cerebraltherapeutika ist bisher eine derartig starke und organspezifische cerebrale Wirkung nicht bekannt geworden, so dass die Verwendung der erfindungsgemässen Verbindung als Cerebraltherapeutikum eine Bereicherung der Pharmazie darstellt.

Die Verbindung 1,4-Dihydro-2,6-dimethyl-4-(3-nitrophenyl)-pyri-din-3-β-methoxyethylester-5-isopropylester (Kurzbezeichnung: Bay e 9736 oder Nimodipin) wird in an sich bekannter Weise erhalten, indem man z.B. 3-Nitrobenzylidenacetessigsäure-β-methoxyethylester mit β-Aminocrotonsäureiso-propylester in einem inerten organischen Lösungsmittel bei erhöhter Temperatur umsetzt. Die Verbindung besitzt einen Schmelzpunkt von 125°C.

Weitere mögliche Herstellungsverfahren sind in der DT-OS 2 117 571 und DT-OS 2 117 573 beschrieben.

Die vorteilhafte organspezifische Wirkung der erfindungsgemässen Verbindung ist gekoppelt mit einer sehr guten Verträglichkeit und einer lang anhaltenden Wirkungsdauer. Ein Vergleich mit der in der DT-OS 2 407 115 als besonders wirksam hervorgehobenen Verbindung 2,6-Dimethyl-4-(3-nitrophenyl-1,4-dihydropyridin-3,5-dicarbonsäure-3-β-(N-benzyl-N-methylamino)-äthylester-5-methylester zeigt, dass die erfindungsgemässe Verbindung nicht nur eine deutlich stärkere cerebrale Mehrdurchblutung verursacht, sondern neben der grösseren Wirkungsdauer auch ein vorteilhafteres Wirkungsprofil besitzt.

Die überraschenden vorteilhaften Eigenschaften seien durch folgende Untersuchungen belegt.

An Hunden, Katzen, Ratten, Kaninchen, Rhesus- und Totenkopfaffen erhöht die erfindungsgemässe Verbindung die Gehirndurchblutung nach enteraler und parenteraler Applikation in einem breiten Dosenbereich. Die cerebral-vasodilatierende Wirkung steht im Vordergrund des Wirkungsspektrums der Substanz: sie erfolgt nach niedriger Dosierung und sehr spezifisch in den cerebralen Gefässen. Mit dieser überraschenden Prädilektivität der cerebrovaskulären Wirkung un-terscheidet sich die erfindungsgemässe Verbin-dung deutlich von sämtlichen handelsüblichen Präparaten dieser Kategorie, sowie auch von anderen Dihydropyridin-Derivaten, bei denen auch eine gewisse cerebraldilatierende Wirkung im Tierexperiment deklariert wurde (vergl. DT-OS 2 407 115; YC-93).

Mit der erfindungsgemässen Verbindung können effektive Prophylaxe und Therapie von Folgen eines ischämischen cerebralen Insultes erfolgreich durchgeführt werden. So wird z.B. bei Kat-zen eine postischämische cerebrale Minder-durchblutung völlig verhindert, die postischämi-sche EEG-Veränderungen deutlich verbessert und die Mortalität nach einem experimentellen cerebralen Isult drastisch reduziert.

Mit dieser cerebralen antiischämischen Wirkung ist die erfindungsgemässe Verbindung bislang konkurrenzlos. Es ist mit keinem der existierenden Pharmaka dieser Kategorie gelungen, eine derartige Wirkung zu erreichen.

Bei Ratten und Mäusen sind die experimentell induzierten Lern- und Gedächtnisstörungen vaskulärer und nichtvaskulärer Genese (cerebrale Ischämie, Hypoxie, Konvulsionen, Schlafentzug u.a.) durch Behandlung mit der erfindungsgemässen Verbindung überraschenderweise deutlich verbessert bis völlig normalisiert. Mit dem Ausmass dieser Wirkung ist die Verbindung den handelsüblichen Präparaten weit überlegen.

Die überraschende vorteilhafte Wirkung der erfindungsgemässen Verbindung sei durch die Daten der folgenden Tabelle beispielhaft belegt.

Tabelle: Wirkung von Substanzen auf die Durchströmung der cerebralen und extracerebralen Gefässe bei Hunden

| Substanz | Dosis mg/kg i.v. | Anzahl der Tiere | Steigerung der Gehirndurchblutung ([133]Xenon-Clearance) | Durchströmungssteigerung A. carotis ext. (el.-magn. Flowmeter) | A. femoralis (el.-magn. Flowmeter) |
|---|---|---|---|---|---|
| BAY e 9736 | 0,01 | 22 | 30% | 26% | 0,5% |
| YC 93 | 0,01 | 3 | 16% | 40% | 10,0% |
| Bencyclan (Fludilat[ir]) | 10,0 | 5 | 34% | – | 152% |
| Cinnarizin (Stutgeron[ii]) | 10,0 | 4 | 43% | – | 65% |

Die Verbindung kann in bekannter Weise in die üblichen Formulierungen übergeführt werden, wie Tabletten, Kapseln, Dragees, Pillen, Suppositorien, Granulate, Aerosole, Sirupe, flüssige, feste und cremeartige Emulsionen und Suspensionen und Lösungen, unter Verwendung inerter, nichttoxischer, pharmazeutisch geeigneter Trägerstoffe oder Lösungsmittel. Hierbei soll die therapeutisch wirksame Verbindung jeweils in einer Konzentration von etwa 0,1 bis 90 Gewichtsprozent der Gesamtmischung vorhanden sein, d.h. in Mengen, die ausreichend sind, um den angegebenen Dosierungsspielraum zu erreichen.

Die ausgesprochen gute Resorption der erfindungsgemässen Verbindung durch die Schleimhäute des gastrointestinalen Traktes ermöglicht die Anwendung von vielen galenischen Zubereitungen: die Substanz kann als Medikament in Form von Dragees, Kapseln, Beisskapseln, Tabletten, Tropfen, Syrup aber auch Zäpfchen und sogar Salben angewendet werden.

Die Formulierungen werden beispielsweise hergestellt durch Verstrecken des Wirkstoffs mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nichttoxische organische Lösungsmittel, wie Paraffine (z.B. Erdölfraktionen), pflanzliche Öle (z.B. Erdnuss-Sesamöl), Alkohole (z.B. Äthylalkohol, Glycerin), Glykole (z.B. Propylenglykol, Polyäthylenglykol); feste Trägerstoffe, wie z.B. natürliche Gesteinsmehle (z.B. hochdisperse Kieselsäure, Silikate), Zucker (z.B. Roh-, Milch- und Traubenzucker); Emulgiermittel, wie nichtionogene und antionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise enteral oder parenteral, insbesondere oral.

Im Falle der enteralen Anwendung können Tabletten selbstverständlich ausser den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, kann der Wirkstoff ausser mit den obengenannten Hilfsstoffen mit verschiedenen Geschmacksaufbessern oder Farbstoffen versetzt werden.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,0001 bis 0,5 mg/kg, vorzugsweise etwa 0,001 bis 0,1 mg/kg Körpergewicht pro Tag zur Erzielung wirksamer Ergebnisse zu verabreichen, und bei enteraler Applikation beträgt die Dosierung etwa 0,001 bis 1 mg/kg, vorzugsweise 0,01 bis 0,5 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch auf Grund der Spezies und deren individuellem Verhalten gegenüber dem Medikament bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Fall der Applikation grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Beispiele für pharmazeutische Formulierungen:

1. Weichgelatinekapseln mit 5 mg Wirkstoff pro Kapsel: Für ca. 10 000 Kapseln wird eine Lösung folgender Zusammensetzung hergestellt:

| | |
|---|---|
| Bay e 9736 Wirkstoff | 58,8 g |
| Glycerin | 240,0 g |
| Polyäthylenglykol 400 | 3833,2 g |
| Wasser | 400,0 g |
| | 4532,0 g |

Die Lösung wird nach an sich bekannter Weise in Weichgelatinekapseln oblong der Grösse 6 minims abgefüllt. Die Kapseln sind zum Zerbeissen oder zum Schlucken geeignet.

2. Tabletten, lackierte Tabletten oder Dragees mit 10 mg Wirkstoff:

Die folgenden Mengen beziehen sich auf Herstellung von 100 000 Tabletten bzw. Kernen:

| | |
|---|---|
| Bay e 9736 fein gemahlener Wirkstoff | 1,00 kg |
| Milchzucker | 10,25 kg |
| Stärke | 2,70 kg |
| Mikrokristalline Cellulose | 2,70 kg |

Obige Bestandteile werden in einem Planetenmischer gemischt und anschliessend mit einer Lösung, hergestellt aus

| | |
|---|---|
| Polyvinylpyrrolidon (Mol. Gewicht z.B. 25 000) | 1,20 kg |
| Polysorbate 80 USP (Tween 80*) und | 0,06 kg |
| Wasser | ca. 4,00 kg |

gemischt und in an sich bekannter Weise granuliert, indem die feuchte Masse geraspelt und getrocknet wird. Anschliessend gibt man zu:

| | |
|---|---|
| Magnesiumstearat | 0,09 kg |

Die fertige Tablettenmischung von 18 kg wird zu gewölbten Tabletten von 180 mg Gewicht verpresst. Durchmesser der Tabletten 8 mm.

Die Tabletten können nach an sich bekannter Weise lackiert oder dragiert werden.

3. Tropfen mit 4 mg Wirkstoff pro ml:
Folgende Lösung wird hergestellt

Für Tropfen mit 4 mg pro ml

| | |
|---|---|
| Bay e 9736 Wirkstoff | 4,0 g |
| Äthanol 96%ig | 450,0 g |
| Aroma flüssig | 6,0 g |
| Methylparaben | 1,0 g |
| Polyäthylenglykol 400 | 50,0 g |
| 50%iger Zuckersirup | 400,0 g |
| Lebensmittelfarbstoff (Gelborange S) | 0,6 g |
| Wasser ad | 1000,0 ml |

Der Wirkstoff, Methylparaben und Aroma werden bei Zimmertemperatur gelöst. Dann gibt man Polyäthylenglykol 400 und den 50%igen Zuckersirup langsam unter Rühren zu, löst den Farbstoff und ergänzt mit Wasser auf 1000 ml.

Die Lösung wird in braune Flaschen abgefüllt, wobei gegebenenfalls noch Süssstoffe zugesetzt werden können.

4. Sirup mit 10 mg Wirkstoff pro 10 ml:

| | |
|---|---|
| Bay e 9736 Wirkstoff | 1,0 g |
| Methylparaben | 1,0 g |
| Äthanol 96%ig | 250,0 g |
| Aroma flüssig | 4,0 g |
| Polyäthylenglykol 400 | 100,0 g |
| Glyzerin | 250,0 g |
| 50%iger Zuckersirup | 300,0 g |
| Lebensmittelfarbstoff (Gelborange S) | 0,5 g |
| Wasser ad | 1000,0 ml |

Die Herstellung erfolgt in analoger Weise zu dem Beispiel 3.

**Patentansprüche**

1. Verwendung von
1,4-Dihydro-2,6-dimethyl-4-(3-nitrophenyl)-pyridin-3-β-methoxy-ethylester-5-isopropylester
zur Herstellung von cerebral wirksamen Arzneimitteln.

2. Verwendung von
1,4-Dihydro-2,6-dimethyl-4-(3-nitrophenyl)-pyridin-3-β-methoxy-ethylester-5-isopropylester
und mikrokristalliner Cellulose zur Herstellung von cerebral wirksamen Arzneimitteln.

3. Verwendung von
1,4-Dihydro-2,6-dimethyl-4-(3-nitrophenyl)-pyridin-3-β-methoxy-ethylester-5-isopropylester
und mikrokristalliner Cellulose zur Herstellung von cerebral wirksamen Arzneimitteln in fester Zubereitungsform.

**Claims**

1. Use of
1,4-dihydro-2,6-dimethyl-4-(3-nitrophenyl)-pyridine-3-β-methoxy-ethyl ester 5-isopropyl ester
for the preparation of medicaments with cerebral action.

2. Use of
1,4-dihydro-2,6-dimethyl-4-(3-nitrophenyl)-pyridine-3-β-methoxy-ethyl ester 5-isopropyl ester
and microcrystalline cellulose for the preparation of medicaments with cerebral action.

3. Use of
1,4-dihydro-2,6-dimethyl-4-(3-nitrophenyl)-pyridine-3-β-methoxy-ethyl ester 5-isopropyl ester
and microcrystalline cellulose for the preparation of medicaments with cerebral action which are in the form of solid formulations.

**Revendications**

1. Utilisation de l'ester
3-β-méthoxyéthylique-5-isopropylique de la 1,4-dihydro-2,6-diméthyl-4-(3-nitrophényl)-pyridine
pour la préparation de médicaments actifs sur le cerveau.

2. Utilisation de l'ester
3-β-méthoxyéthylique-5-isopropylique de la 1,4-dihydro-2,6-diméthyl-4-(3-nitrophényl)-pyridine
et de cellulose microacristalline pour la préparation de médicaments actifs sur le cerveau.

3. Utilisation de l'ester
3-β-méthoxyéthylique-5-isopropylique de la 1,4-dihydro-2,6-diméthyl-4-(3-nitrophényl)-pyridine
et de la cellulose microcristalline pour la préparation de médicaments actifs sur le cerveau à l'état de composition solide.